# EUROPEAN PATENT SPECIFICATION

(11) **EP 1 359 920 B1**
(45) Date of publication and mention of the grant of the patent: **22.08.2007**
(21) Application number: 01992297.0
(22) Date of filing: 21.12.2001
(51) Int. Cl.: A61K 31/565, A61K 31/57, A61K 45/06, A61P 15/12

(54) **USES OF ORAL FORMULATIONS FOR THE TREATMENT OF FEMALE SEXUAL DYSFUNCTION**
VERWENDUNG ORALER FORMULIERUNGEN ZUR BEHANDLUNG DER WEIBLICHEN SEXUELLEN STÖRUNG
UTILISATIONS DES FORMULATIONS ORALES POUR LE TRAITEMENT DEs DYSFONCTIONNEMENTS SEXUELS CHEZ LA FEMME

(30) Priority: 22.12.2000 US 257745 P
(43) Date of publication of application: 12.11.2003
(73) Proprietor: Barr Laboratories, Inc., Woodcliff Lake, New Jersey 07677 (US)
(72) Inventor: LEONARD, Thomas, W., Wilmington, NC 28405 (US); WALDON, R., Forrest, Wilmington, NC28409 (US)
(74) Representative: Williams, Richard Andrew Norman
(86) International application number: PCT/US2001/049978
(87) International publication number: WO 2002/051420

(56) References cited:
- WO-A-99/21562
- US-A- 4 755 386
- US-A- 5 565 466
- US-A- 6 036 977
- US-A- 6 117 446

## Description

**Field of the Invention**

The present invention generally relates to pharmaceutical formulations for the treatment of female sexual dysfunction, along with methods of administering the same.

**Background of the Invention**

Female sexual dysfunction is a complex medical condition involving the interplay of psychological, hormonal, and physiological factors. Hormonal aberrations can have an adverse effect on female sexual function as a result of one or more aspects of female physiology. For example, estrogen levels are related to such aspects of female sexuality including overall physiological health of the vagina, integrity of the vaginal epithelium, and the ability of the vagina to produce sufficient lubrication. Substantial decreases in circulating levels of estrogens naturally occur during menopause. Menopause, which typically occurs during middle age, results in ovarian shutdown, and commensurate therewith is a profound decrease in circulating levels of estrogens.

Ovarian shutdown also causes aberrations in androgen levels. The decline in androgen levels has potential impact in several post-menopausal areas in women. One aspect of androgen effects in females relates to sexual function, including effects on desire, sensitivity to stimulation, ability to experience fantasy and the ability to reach orgasm. Circulating levels of androgens typically decrease several years after the onset of decreases in estrogen levels due to natural menopause. In surgical menopause, the decrease in circulatory levels of androgens mimics that of estrogens and is precipitous. The combination of estrogen and androgen treatment has been suggested by Shifren et al., The New England Journal of Medicine, 343:682-688 (2000). Shifren et al., proposes transdermal administration of testosterone in women who had impaired sexual function after surgically induced menopause. Similarly, U.S. Patent No. 6,117,446 and 6,284,263 to Place proposes a buccal dosage unit comprising progestin, an estrogen, and optionally an androgenic agent

Another aspect of female sexual dysfunction is aberrations in the clitoral erectile response. Such response is an interplay of the autonomic nervous system, endocrine system and the circulatory system. Although the failure of erectile response (impotence) is most commonly associated with men, it is also an aspect of female sexual dysfunction. Increased blood flow is necessary for a clitoral erection along with vaginal engorgement. Regular stimulation of clitoral and vaginal blood flow is necessary for healthy and functional vaginal and clitoral tissue. Also there is evidence that blood flow is related to the achievement of orgasm in women, although the relationship is not as clear as it is for men. It has been suggested that the addition of vasodilators can be used to improve female sexual response. For example, U.S. Patent Nos. 5,565,466 to Gioco et al., 6,051,594 to Lowrey and 6,011,043 to Estok suggest the use of phentolamine. U.S. Patent Nos. 5,891,915 to Wysor et al. and 6,046,240 to See propose the administering of prostaglandin E. Additionally, other patents issued to Place et al. including U.S. Patent No. 6,294,550 and U.S. Patent No. 6,306,841 provide methods of treating a sexual dysfunction in a female individual by administering a vasoactive agent to the vagina, vulval area or urethra of the individual undergoing treatment.

U.S. Patent No. 4 755 386 discloses a buccal composition containing an estrogen, an androgen and verapamil which is known to be a vasodilation compound.

WO 99/21562 discloses pharmaceutical compositions for the treatment of female sexual dysfunction containing a vasoactive agent e.g. α-blockers like phentolamine, an androgen and as well an estrogen.

Thus, it would be desirable to provide methods and formulations for treatment of female sexual dysfunction that addresses the various psychological, hormonal and physiological factors in such a complex medical condition.

**Summary of the Invention**

The present invention provides for the use of an estrogenic compound, an androgenic compound and a vasodilation compound for the manufacture of an oral medicament for treatment of female sexual dysfunction.

The invention also provides for use of an estrogenic compound, an androgenic compound and a vasodilation compound for the manufacture of medicaments for the treatment of female sexual dysfunction, wherein the estrogenic compound and androgenic compound are combined as a first oral medicament and the vasodilation compound is provided as a second medicament. Preferably the vasodilation compound is administered on a performance-on-demand basis. In an alternative embodiment of the invention, the combination of the estrogenic compound and the androgenic compound is administered on a chronic basis, and the vasodilation compound is administered on a chronic basis. Alternatively the combination of the estrogenic compound and the androgenic compound, and the vasodilation compound are administered simultaneously.

In another embodiment of the invention, the combination of the estrogenic compound and the androgenic compound, and the vasodilation compound is to be administered in tablet form.

In yet another embodiment of the invention, the combination of the estrogenic compound and the androgenic compound is to be administered on a chronic basis and the vasodilation compound is to be administered on a performance-on-demand basis. Preferably the vasodilation compound is to be administered before or during sexual activity.

In one embodiment of the invention the combination of the estrogenic compound and the androgenic compound is to be administered in a tablet form, and the vasodilation compound is to be administered in a tablet form. Alternatively the combination of the estrogenic compound and androgenic compound is to be administered in a tablet form, and the vasodilation compound is to be administered in a topical form. Preferably the topical form is selected from the group consisting of patches, gels, and creams. Preferably the topical form is applied to the skin. More preferably the topical form is applied to the vagina.

Preferably the estrogenic compound is selected from the group consisting of estrone, 17α-estradiol, 17β-estradiol, equilin, 17α-dihydroequilin, 17β-dihydroequilin, equilenin, 17α-dihydroequilenin, 17β-dihydroequilenin, Δ^{8,9}-dehydroestrone, 17*α-Δ*^{8,9}-dehydroestradiol, 17β-Δ^{8,9} -dehydroestradiol, ethinyl estradiol, estradiol valerate, 6-OH equilenin, 6-OH 17α-dihydroequilenin, 6-OH 17β-dihydroequilenin, and mixtures, conjugates and salts thereof.

Preferably the androgenic compound is selected from the group consisting of methyltestosterone, androsterone, androsterone acetate, androstenedione, androstenediol, androsterone propionate, androsterone benzoate, androsteronediol, androsteronediol-3-acetate, androsteronediol-17-acetate, androsteronediol 3,17-diacetate, androsteronediol-17-benzoate, androsteronediol-3-acetate-17-benzoate, androsteronedione, dehydroepiandrosterone, sodium dehydroepiandrosterone sulfate, dromostanolone, dromostanolone propionate, ethylestrenol, fluoxymesterone, derdicale, nandrolone phenpropionate, nandrolone decanoate, nandrolone furylpropionate, nandrolone cyclohexane-propionate, nandrolone benzoate, nandrolone cyclohexanecarboxylate, oxandrolone, oxymetholone, stanozolol, testosterone, 17α-methyl-19-nortestosterone, testosterone decanoate, 4-dihydrotestosterone, 5α-dihydrotestosterone, testolactone, pharmaceutically acceptable esters and salts thereof, and combinations of any of the foregoing.

In one embodiment of the invention, the vasodilation compound is an alpha andrenergic antagonist. Preferably the vasodilation compound is phentolamine mesylate or phentolalmine hydrochloride. Preferably the vasodilation compound further includes apomorphine. More preferably the medicament further comprises a progestin compound, preferably wherein the progestin compound is selected from the group consisting of desogestrel, dydrogesterone, ethynodiol diacetate, medroxyprogesterone, levonorgestrel, medroxyprogesterone acetate, hydroxyprogesterone caproate, norethindrone, norethindrone acetate, norethynodrel, allylestrenol, 19-nortestosterone, lynoestrenol, quingestanol acetate, medrogestone, norgestrienone, dimethisterone, ethisterone, cyproterone acetate, chlormadinone acetate, megestrol acetate, norgestimate, norgestrel, desogestrel, trimegestone, gestodene, nomegestrol acetate, nomegestrol progesterone, 5α-pregnan-3β,20α-diol sulfate, 5α-pregnan-3β, 20β-diol sulfate, 5α-pregnan-3β-ol-20-one, 16,5α-pregnen-3β-ol-20-one,4-pregnen-20β-ol-3-one-20-sulfate, acetoxypregnenolone, anagestone acetate, cyproterone, dihydrogesterone, flurogestone acetate, gestadene, hydroxyprogesterone actetate, hydroxymethylprogesterone, hydroxymethyl progesterone acetate, 3-ketodesogestrel, megestrol, melengestrol acetate, norethisterone and mixtures thereof.

In one embodiment of the invention, the medicament is formulated to provide quick, sustained or delayed release of an active ingredient. Alternatively the medicament provides the vasodilation compound on an immediate release basis and the estrogenic compound and the androgenic compound on a modified release basis. Preferably the medicament is for the treatment of female sexual dysfunction in a post-menopausal female.

A pharmaceutical composition for the treatment of female sexual dysfunction, particularly that experienced by post menopausal females, is provided. The composition comprises a therapeutically effective amount of an estrogenic compound, androgenic compound, vasodilation compound, and a pharmaceutically acceptable carrier. For women with an intact uterus, an appropriate amount of progestin may optionally be included. In pre-menopausal women, particularly those using oral contraceptives, positive results may be achieved without the use of estrogens.

Also described is the use of an estrogenic compound, androgenic compound, vasodilation compound, and a pharmaceutically acceptable carrier for the treatment of female sexual dysfunction . The vasodilator can be continuously administered (on a daily basis) or can be a sequential therapy (prior to sex).

**Detailed Description of the Preferred Embodiments**

The invention will now be described with reference to the embodiments set forth herein. These embodiments are intended to illustrate the invention and are not meant to limit the scope of the invention, which is defined by the claims.

A pharmaceutical composition comprises a therapeutically effective amount of an estrogenic compound, androgenic compound, vasodilation compound, and a pharmaceutically acceptable carrier. A "therapeutically effective" amount as used herein is an amount of an estrogenic compound, androgenic compound and vasodilation compound that is sufficient to treat (*e.g*. increase, boost, augment) sexual function in a subject. The therapeutically effective amount will vary with the age and physical condition of the patient, the severity of the treatment, the duration of the treatment, the nature of any concurrent treatment, the pharmaceutically acceptable carrier used and like factors within the knowledge and expertise of those skilled in the art. Pharmaceutically acceptable carriers are preferably liquid, particularly aqueous, carriers, the selection of which are known in the art.

Estrogen levels are related to the general physiological health of the vagina, provide vasodilation effects and stimulate mucous production. Suitable estrogenic compounds include estrone, 17α-estradiol, 17β-estradiol, equilin, 17α-dmydioequilin, 17β-dihydroequilin, equilenin. 17α-dihydroequilenin, 17β-dihydroequilenin, Δ^{8,9}-dehydroestrone, 17α-Δ^{8,9}-dehydroestiadiol, 17β-Δ^{8,9}-dehydroestradiol, ethinyl estradiol, estradiol valerate, 6-OH equilenin, 6-OH 17α-dihydroequilenin, 6-OH 17β-dihydroequilenin, and mixtures, conjugates and salts thereof, and the estrogen ketones and their corresponding 17α- and 17-β hydroxy derivatives. Conjugates may be formed by those skilled in the art, including, but not limited to, sulfate and glucuronide. The most preferred estrogen conjugates are estrogen sulfates. The estrogenic compounds may also be present as salts of estrogens conjugates. The salts may be various salts understood by those skilled in the art, including, but not limited to, sodium salts, calcium salts, magnesium salts, lithium salts, potassium salts and piperazine salts. The most preferred salts are sodium salts. The estrogenic compounds can be derived from natural and synthetic sources. Preferably, the therapeutically effective amount of estrogenic compound is equivalent potency of about 0.0 to about 3 mg, and preferably about 0.25 to about 2 mg of estradiol administered orally. Estrogens may not be necessary in premenopausal women, particularly those using oral contraceptives.

With respect to androgenic compounds, androgen levels are related to desire, sensitivity to stimulation, ability to experience fantasy and ability to reach orgasm. Therapeutic effects are more closely related to unbound circulatory levels of androgens as compared to total circulating androgens. As androgens are primarily bound to sex hormone binding globulin (SHBG), factors that increase the level of SHBG can decrease unbound androgen levels and impact sexuality. Notably, treatment of women with estrogens to correct deficiency or to impact fertility (oral contraceptives) increases levels of SHBG causing effective circulating levels of androgens to decrease thereby impacting the sexual function of women normally having adequate levels of circulating androgens.

Suitable androgenic compounds include methyltestosterone, androsterone, androsterone acetate, androsterone propionate, androsterone benzoate, androsteronediol, androsteronediol-3-acetate, androsteronediol-17-acetate, androsteronediol 3-17-diacetate, androsteronediol-17-benzoate, androsteronedione, androstenedione, androstenediol, dehydroepiandrosterone, sodium dehydroepiandrosterone sulfate, dromostanolone, dromostanolone propionate, ethylestrenol, fluoxymesterone, nandrolone phenpropionate, nandrolone decanoate, nandrolone furylpropionate, nandrolone cyclohexane-propionate, nandrolone benzoate, nandrolone cyclohexanecarboxylate, androsteronediol-3-acetate-17-benzoate, oxandrolone, oxymetholone, stanozolol, testosterone, testosterone decanoate, 4-dihydrotestosterone, 5α-dihydrotestosterone, testolactone, 17α-methyl-19-nortestosterone and pharmaceutically acceptable esters and salts thereof, and combinations of any of the foregoing. Preferably the therapeutically effective amount of the androgenic compound is equivalent to oral doses of about 0.15 to about 5 mg of methyl testosterone. Vasodilation compounds or agents facilitate the clitoral erectile response in females as a result of engorgement of the erectile tissues of the genitalia with blood in response to sexual stimulation. Suitable vasodilation compounds include alpha adrenergic antagonists. Exemplary α-adrenergic compounds include phentolamine, phenoxybenzalamine, tolazoline, doxazosin, dibenamine, prazosin, prazosin hydrochloride, phenoxybenzamine and the like. Preferably, phentolamine is used and can form pharmaceutically acceptable salts with organic and inorganic acids, such as described in U.S. Patent No. 6,001,845 to Estok. Preferably phentolamine mesylate or phentolamine hydrochloride is used. Optionally apomorphine or other opiate derivatives may be used with phentolamine. Other vasodilation compounds include phosphodiesterase type 5 inhibitors (e.g., suldenafi), prostaglandin E compounds (e.g., alprostodil), thymoxamine, bromocriptine, yohimbine, paperverine, organic nitrates, imipramine, verapamil, naftidrofuryl, and isoxsuprine. Combinations of the various vasodilation compounds may be used. Preferably, the therapeutically effective amount for oral use of vasodilation compound is equivalent to doses of about 5 to about 80 mg, and preferably about 20 about 80 mg of phentalomine hydrochloride or mesylate.

For women with an intact uterus, a progestin compound may optionally be included. Suitable progestin compounds include desogestrel dydrogesterone, ethynodiol diacetate, medroxyprogesterone, levonorgestrel, medroxyprogesterone acetate, hydroxyprogesterone caproate, norethindrone, norethindrone acetate, norethynodrel, allylestrenol, 19-nortestosterone, lynoestrenol, quingestanol acetate, medrogestone, norgestrienone, dimethisterone, ethisterone, cyproterone acetate, chlormadinone acetate, megestrol acetate, norgestimate, norgestrel, desogrestrel, trimegestone, gestodene, nomegestrol acetate, progesterone, 5α-pregnan-3β, 20α-diol sulfate, 5α-prepan-3β,20β-diol sulfate, 5α-pregnan-3β-ol-20-one, 16, 5α-pregnen-3β-ol-20-one, 4-pregnen-20β-ol-3-one-20-sulfate, acetoxypregnenolone, anagestone acetate, cyproterone, dihydrogesterone, flurogestone acetate, gestadene, hydroxyprogesterone acetate, hydroxymethylprogesterone, hydroxymethyl progesterone acetate, 3-ketodesogestrel, megestrol melengestrol, acetate, norethisterone and mixtures thereof. Preferably the therapeutically effective oral amount is equivalent of doses of about 0.5 mg to about 20 mg, and preferably about 1.0 to about 2.5 mg of medroxyprogesterone acetate.

The present invention encompasses pharmaceutically acceptable drug products comprising a composition of matter of the present invention and at least one pharmaceutically acceptable carrier, diluent, or excipient, the selection of which are known to the skilled artisan. Also described are drug product formulations, for example, in the form of tablets; effervescent tablets; pills; powders; elixirs; suspensions; emulsions; solutions; syrups; soft and hard gelatin capsules; transdermal patches; topical gels, creams and the like; vaginal suppositories; sterile injectable solutions; and sterile packaged powders, sublingual tablets, buccal tablets, buccal adhesive systems.

In certain embodiments, the drug product is present in a solid pharmaceutical composition suitable for oral administration. A solid composition of matter according to the present invention may be formed and may be mixed with and/or diluted by excipients. The solid composition of matter may also be enclosed within a carrier which may be, for example, in the form of a capsule, sachet, tablet, paper, or other container. When the excipient serves as a diluent, it may be a solid, semi-solid, or liquid material which acts as a vehicle, carrier, or medium for the composition of matter.

Various suitable excipients will be understood by those skilled in the art and may be found in the National Formulary, 19: 2404-2406 (2000), pages 2404 to 2406. Examples of suitable excipients include, but are not limited to, starches, gum arabic, calcium silicate, microcrystalline cellulose, methacrylates, shellac, polyvinylpyrrolidone, cellulose, water, syrup, and methylcellulose. The drug product formulations can additionally include lubricating agents such as, for example, talc, magnesium stearate and mineral oil; wetting agents; emulsifying and suspending agents; preserving agents such as methyl- and propyl hydroxybenzoates; sweetening agents; or flavoring agents. Polyols, buffers, and inert fillers may also be used. Examples of polyols include, but are not limited to, mannitol, sorbitol, xylitol, sucrose, maltose, glucose, lactose, dextrose, and the like. Suitable buffers encompass, but are not limited to, phosphate, citrate, tartarate, succinate, and the like. Other inert fillers that may be used encompass those which are known in the art and are useful in the manufacture of various dosage forms. If desired, the solid formulations may include other components such as bulking agents and/or granulating agents, and the like. The drug products of the invention may be formulated so as to provide quick, sustained, or delayed release of the active ingredient after administration to the patient by employing procedures well known in the art.

To form tablets for oral administration, the composition of matter of the present invention may be made by a direct compression process. In this process, the active drug ingredients may be mixed with a solid, pulverant carrier such as; for example, lactose, saccharose, sorbitol, mannitol, starch, amylopectin, cellulose derivatives or gelatin, and mixtures thereof, as well as with an antifriction agent such as, for example, magnesium stearate, calcium stearate, and polyethylene glycol waxes. The mixture may then be pressed into tablets using a machine with the appropriate punches and dies to obtain the desired tablet size. The operating parameters of the machine may be selected by the skilled artisan. Alternatively, tablets for oral administration may be formed by a wet granulation process. Active drug ingredients may be mixed with excipients and/or diluents. The solid substances may be ground or sieved to a desired particle size. A binding agent may be added to the drug. The binding agent may be suspended and homogenized in a suitable solvent. The active ingredient and auxiliary agents may also be mixed with the binding agent solution. The resulting dry mixture is moistened with the solution uniformly. The moistening typically causes the particles to aggregate slightly, and the resulting mass is pressed through a stainless steel sieve having a desired size. The mixture is then dried in controlled drying units for the determined length of time necessary to achieve a desired particle size and consistency. The granules of the dried mixture are sieved to remove any powder. To this mixture, disintegrating; antifriction, and/or anti-adhesive agents are added. Finally, the mixture is pressed into tablets using a machine with the appropriate punches and dies to obtain the desired tablet size. The operating parameters of the machine may be selected by the skilled artisan.

If coated tablets are desired, the above-prepared cores may be coated with a concentrated solution of sugar or cellulosic polymers, which may contain gum arabic, gelatin, talc, titanium dioxide, or with a lacquer dissolved in a volatile organic solvent, aqueous solvent, or a mixture of solvents. To this coating, various dyes may be.added in order to distinguish among tablets with different active compounds or with different amounts of the active compound present In a particular embodiment, the active ingredient may be present in a core surrounded by one or more layers including enteric coating layers.

Soft gelatin capsules may be prepared in which capsules contain a mixture of the active ingredient and vegetable oil. Hard gelatin capsules may contain granules of the active ingredient in combination with a solid, pulverulent carrier, such as, for example, lactose, saccharose, sorbitol, mannitol, potato starch, corn starch, amylopectin, cellulose derivatives, and/or gelatin.

In one preferred embodiment, the formulation is in the form of orally-administered tablets which contain the composition of matter of the present invention as set forth herein along with the following inactive ingredients: calcium phosphate tribasic, calcium sulfate, carnauba wax, cellulose, glyceryl monooleate, lactose, magnesium stearate, methyl cellulose, pharmaceutical glaze, polyethylene glycol, stearic acid, sucrose, and titanium dioxide. Such ingredients may be present in amounts similar to those present in Premarin® (conjugated estrogens tablets, USP) made commercially available by Wyeth-Ayerst Laboratories of Philadelphia, Pennsylvania. Tablets employing the active ingredients of the invention may contain excipients similar to those contained in the 0.3 mg, 0.625 mg, and 1.25 mg tablets of Premarin® (conjugated estrogens tablets, USP).

Liquid preparations for oral administration may be prepared in the form of syrups or suspensions, e.g., solutions containing an active ingredient, sugar, and a mixture of ethanol, water, glycerol, and propylene glycol. If desired, such liquid preparations may contain coloring agents, flavoring agents, and saccharin. Thickening agents such as carboxymethylcellulose may also be used.

Also described are the above formulations to be used for parenteral administration, such a formulation may comprise sterile aqueous injection solutions, non-aqueous injection solutions, or both comprising the composition of matter of the present invention. When aqueous injection solutions are prepared, the composition of matter may be present as a water soluble pharmaceutically acceptable salt. Parenteral preparations may contain anti-oxidants, buffers, bacteriostats, and solutes which render the formulation isotonic with the blood of the intended recipient. Aqueous and non-aqueous sterile suspensions may include suspending agents and thickening agents. The formulations may be presented in unit-dose or multi-dose containers, for example sealed ampules and vials. Extemporaneous injection solutions and suspensions may be prepared from sterile powders, granules and tablets of the kind previously described.

Also described is the drug product of the present invention in the form of an injectable solution containing a predetermined amount (e.g., 25 mg) of the composition of matter in a sterile lyphilized cake which also contains lactose, sodium citrate, and simethicone. The pH of a solution containing the above ingredients may be adjusted using a suitable buffer (e.g., sodium hydroxide or hydrochloric acid). Reconstitution may be carried out according to known methods, e.g., using a sterile diluent (5 mL) containing 2 percent by volume benzyl alcohol in sterile water. A preferred injectable solution is similar to Premarin^{®} Intravenous made commercially available by Wyeth-Ayerst Laboratories.

The composition of matter also may be formulated such that it is suitable for topical administration (e.g., vaginal cream). These formulations may contain various excipients known to those skilled in the art. Suitable excipients may include, but are not limited to, cetyl esters, wax, cetyl alcohol, white wax, glyceryl monostearate, propylene glycol, monostearate, methyl stearate, benzyl alcohol, sodium lauryl sulfate, glycerin, mineral oil, water, carbomer, ethyl alcohol; acrylate adhesives, polyisobufylene adhesives, and silicone adhesives.

Also described is the drug product in the form of a vaginal cream containing the composition of matter as set forth herein present in a nonliquefying base. The nonliquefying base may contain various inactive ingredients such as, for example, cetyl esters wax, cetyl alcohol, white wax, glyceryl monostearate, propylene glycol, monostearate, methyl stearate; benzyl alcohol, sodium lauryl sulfate, glycerin, and mineral oil. Such composition may be formulated similar to Premarin^{®} Vaginal Cream made commercially available by Wyeth-Ayerst Laboratories.

Dosage units for vaginal or rectal administration may be prepared in the form of suppositories which may contain the composition of matter in a mixture with a neutral fat base polyethylene glycol, or they may be prepared in the form of gelatin-rectal capsules which contain the active substance in a mixture with a vegetable oil or paraffin oil.

The pharmaceutical compound of the present invention can be administered in the above forms in a variety of manners. For example, a therapeutically effective amount of an estrogenic compound and a therapeutically effective amount of an androgenic compound can be administered to the female, for example, on a chronic basis in tablet form, followed by administering a therapeutically effective amount of a vasodilation compound on a performance-on-demand (POD) basis including before or during sexual activity. Or all the compounds can be administered as a combination, such as in tablet form. The selection of the form of administration will be within the skill of one in the art.

The present invention is explained in greater detail in the Examples which follow. These examples are intended as illustrative of the invention and are not to be taken are limiting thereof.

EXAMPLES

Example 1

Delivery System for Oral Administration of Estrogen, Androgen and Vasoldilator

An immediate release tablet may be produced by combining 0.6 mg of estrogen and 2.5 mg of androgen and then dry blending the components with lactose, calcium carbonate, sodium bicarbonate, alpha-tocopherol, maltodextrin and magnesium stearate to form an immediate release tablet. The immediate release tablet containing the estrogen and androgen is given in conjunction with a rapid dissolve tablet of a vasodilator for the treatment of female sexual dysfunction.

The steps that one needs to produce the immediate release tablet follow. First, 8000 grams of lactose monohydrate should be poured into a 10 cubic foot V type blender. Next 500 grams of calcium carbonate and 500 grams of sodium bicarbonate may be added and then the combination may be blended for 10 -15 minutes.

The next step in the production of the tablet includes adding 250 grams of androgen, such as methyltestosterone, and 60 grams of estrogen, such as estradiol, to the mixture in the V-blender and mixing the combination for 10 -12 minutes.

Next, approximately 390 grams of alpha-tocopherol, 200 grams of maltodextrin and 100 grams of magnesium stearate may be added to the mixture in the V blender and the combination may be blended for 2-3 minutes.

This blend may then be transferred to an appropriate tablet press and compressed to make one hundred thousand cores with a weight of 100 mg each, ready for film coating with Eudragit E^{™} to make an immediate release film coated finished tablet. These tablets are designed to be given once a day.

In the same example, the flash release tablet of vasodilator is produced by adding approximately 500 grams of povidone and 500 grams of low molecular weight hydrolyzed gelatin in a 2 cubic foot V type blender and then blending the combination for 2 to 3 minutes. To this mixture 110 grams of phentolamine may be added and then the mixture may be blended for 5 minutes. Next, approximately 50 grams of poloxamer is added and the combination is blended for 2-3 minutes. This blend may then be transferred to an appropriate tablet press to make 11,600 core tablets weighing 100 mg each. Each core contains approximately 10 mg each of phentolamine. The cores may then be coated with a 1% solution of povidone to make a rapidly dissolving tablet to be taken once a day.

Example 2

In this example a single modified release tablet comprising 0.6 mg of estrogen, 2.5 mg of androgen and 10 mg of vasodilator are given for the treatment of female sexual dysfunction.

The modified release tablet may be produced as follows. In the first step 8000 grams of lactose monohydrate is poured into a 10 cubic foot V type blender. Next 500 grams of calcium carbonate and 500 grams of sodium bicarbonate is added to the lactose and then this material is blended for 10-15 minutes.

Step two includes adding 250 grams of androgen, such as methyltestosterone, and 60 grams of estrogen, such as estradiol, to the mixture in the V-blender and then mixing the combination thereof for 10 -12 minutes.

In step three, approximately 390 grams of alpha-tocopherol, 200 grams of maltodextrin and 100 grams of magnesium stearate may be added to the mixture in the V blender and then blended for 2-3 minutes.

This blend may then be transferred to an appropriate tablet press and compressed to make one hundred thousand cores with a weight of 100 mg each, ready for film coating with Eudragit L^{™} to make an enteric coated modified release film coated core. This core may then be coated with phentolamine in a fluid bed dryer to obtain a.10.mg. /core weight gain. These cores are then coated with a solution of povidone in order to give a 5% weight gain. This finished drug product, when taken for the treatment of female sexual dysfunction, provides phentolamine on an immediate release basis and estradiol and methyltestosterone on a modified release basis.

Example 3

Example 3 illustrates a modified release tablet. This tablet contains approximately 0.6 mg of estrogen, 2.5 mg of androgen and 10 mg of vasodilator. These components may be dry blended with lactose, calcium carbonate, sodium bicarbonate, alpha-tocopherol, maltodextrin and magnesium stearate, and then coated with ethylcellulose to form a modified release tablet for the treatment of female sexual dysfunction.

To produce such a tablet the following steps may be taken. In the first step 7000 grams of lactose monohydrate is poured into a 10 cubic foot V type blender. Next, 500 grams of calcium carbonate and 500 grams of sodium bicarbonate are added to the lactose and then the mixture is blended for 10 -15 minutes.

In step two, approximately 1000 grams phentolamine, 250 grams of androgen, such as methyltestosterone, and 60 grams of estrogen, such as estradiol, may be added to the mixture in the V-blender and mixed for approximately 10 - 12 minutes.

Step three includes adding approximately 390 grams of alpha-tocopherol, 200 grains of maltodextrin and 100 grams of magnesium stearate to the mixture in the V blender and then blending this mixture for 2-3 minutes.

This blend may then be transferred to an appropriate tablet press and compressed to make one hundred thousand cores with a weight of 100 mg each, ready for film coating with ethylcellulose to make a modified release film coated finished tablet. These tablets produced may be given once a day for the treatment of female sexual dysfunction.

## Claims

1. Use of an estrogenic compound, an androgenic compound and a vasodilation compound for the manufacture of an oral medicament for treatment of female sexual dysfunction.

2. Use of an estrogenic compound, an androgenic compound and a vasodilation compound for the manufacture of medicaments for the treatment of female sexual dysfunction, wherein the estrogenic compound and androgenic compound are combined as a first oral medicament and the vasodilation compound is provided as a second medicament.

3. Use according to Claim 2 wherein the vasodilation compound is administered on a performance-on-demand basis.

4. Use according to Claim 2 wherein the combination of the estrogenic compound and the androgenic compound is administered on a chronic basis, and wherein the vasodilation compound is administered on a chronic basis.

5. Use according to Claim 2 wherein the combination of the estrogenic compound and the androgenic compound, and the vasodilation compound are administered simultaneously.

6. Use according to Claim 2 wherein the combination of administering the estrogenic compound and the androgenic compound, and administering the vasodilation compound is administered in tablet form.

7. Use according to Claim 2 wherein the combination of the estrogenic compound and the androgenic compound is administered on a chronic basis and wherein the vasodilation compound is administered on a performance-on-demand basis.

8. Use according to any of Claim 3 or 7 wherein the vasodilation compound is administered before or during sexual activity.

9. Use according to Claim 2 wherein the combination of the estrogenic compound and the androgenic compound is administered in a tablet form, and wherein the vasodilation compound is administered in a tablet form.

10. Use according to Claim 2 wherein the combination of the estrogenic compound and androgenic compound is administered in a tablet form, and wherein the vasodilation compound is administered in a topical form.

11. Use according to Claim 10 wherein the topical form is selected from the group consisting of patches, gels, and creams.

12. Use according to Claim 10 or Claim 11, wherein the topical form is applied to the skin.

13. Use according to any of Claims 10 to 12, wherein the topical form is applied to the vagina.

14. Use according to any of the preceding claims wherein the estrogenic compound is selected from the group consisting of estrone, 17α-estradiol, 17β-estradiol, equilin, 17α-dihydroequilin, 17β-dihydroequilin, equilenin, 17α-dihydroequilenin, 17β-dihydroequilenin, Δ^{8,9}-dehydroestrone, 17α-Δ^{8,9}-dehydroestradiol, 17β-Δ^{8,9}-dehydroestradiol, ethinyl estradiol, estradiol valerate, 6-OH equilenin, 6-OH 17α-dihydroequilenin, 6-OH 17β-dihydroequilenin, and mixtures, conjugates and salts thereof.

15. Use according to any of the preceding claims wherein the androgenic compound is selected from the group consisting of methyltestosterone, androsterone, androsterone acetate, androstenedione, androstenediol, androsterone propionate, androsterone benzoate, androsteronediol, androsteronediol-3-acetate, androsteronediol-17-acetate, androsteronediol 3,17-diacetate, androsteronediol-17-benzoate, androsteronediol-3-acetate-17-benzoate, androsteronedione, dehydroepiandrosterone, sodium dehydroepiandrosterone sulfate, dromostanolone, dromostanolone propionate, ethylestrenol, fluoxymesterone, derdicale, nandrolone phenpropionate, nandrolone decanoate, nandrolone furylpropionate, nandrolone cyclohexane-propionate, nandrolone benzoate, nandrolone cyclohexanecarboxylate, oxandrolone, oxymetholone, stanozolol, testosterone, 17α-methyl-19-nortestosterone, testosterone decanoate, 4-dihydrotestosterone, 5α-dihydrotestosterone, testolactone, pharmaceutically acceptable esters and salts thereof, and combinations of any of the foregoing.

16. Use according to any of the preceding claims wherein the vasodilation compound is an alpha andrenergic antagonist.

17. Use according to Claim 16 wherein the vasodilation compound is phentolamine mesylate or phentolahnine hydrochloride.

18. Use according to any of the preceding claims wherein the vasodilation compound further includes apomorphine.

19. Use according to any of the preceding claims, wherein the medicament further comprises a progestin compound.

20. Use according to Claim 19 wherein the progestin compound is selected from the group consisting of desogestrel, dydrogesterone, ethynodiol diacetate, medroxyprogesterone, levonorgestrel, medroxyprogesterone acetate, hydroxyprogesterone caproate, norethindrone, norethindrone acetate, norethynodrel, allylestrenol, 19-nortestosterone, lynoestrenol, quingestanol acetate, medrogestone, norgestrienone, dimethisterone, ethisterone, cyproterone acetate, chlormadinone acetate, megestrol acetate, norgestimate, norgestrel, desogestrel, trimegestone, gestodene, nomegestrol acetate, nomegestrol progesterone, 5α-pregnan-3β,20α-diol sulfate, 5α-pregnan-3β, 20β-diol sulfate, 5α-pregnan-3β-ol-20-one, 16,5α-pregnen-3β-ol-20-one, 4-pregnen-20β-ol-3-one-20-sulfate, acetoxypregnenolone, anagestone acetate, cyproterone, dihydrogesterone, flurogestone acetate, gestadene, hydroxyprogesterone actetate, hydroxymethylprogesterone, hydroxymethyl progesterone acetate, 3-ketodesogestrel, megestrol, melengestrol acetate, norethisterone and mixtures thereof.

21. Use according to any of the preceding claims wherein the medicament is formulated to provide quick, sustained or delayed release of an active ingredient.

22. Use according to Claim 9 wherein the medicament provides the vasodilation compound on an immediate release basis and the estrogenic compound and the androgenic compound on a modified release basis.

23. Use according to any of the preceding claims wherein the medicament is for the treatment of female sexual dysfunction in a post-menopausal female.

## Patentansprüche

1. Verwendung einer östrogenen Verbindung, einer androgenen Verbindung und einer gefäßerweiternden Verbindung zur Herstellung eines oralen Arzneimittels zur Behandlung von weiblicher sexueller Dysfunktion.

2. Verwendung einer östrogenen Verbindung, einer androgenen Verbindung und einer gefäßerweiternden Verbindung zur Herstellung von Arzneimitteln zur Behandlung von weiblicher sexueller Dysfunktion, wobei die östrogene Verbindung und die androgene Verbindung als ein erstes orales Arzneimittel kombiniert werden und die gefäßerweiternde Verbindung als ein zweites Arzneimittel bereitgestellt wird.

3. Verwendung nach Anspruch 2, wobei die gefäßerweiternde Verbindung auf einer Leistung-bei-Bedarf-Basis verabreicht wird.

4. Verwendung nach Anspruch 2, wobei die Kombination der östrogenen Verbindung und der androgenen Verbindung auf einer ständigen Basis verabreicht wird und wobei die gefäßerweiternde Verbindung auf einer ständigen Basis verabreicht wird.

5. Verwendung nach Anspruch 2, wobei die Kombination der östrogenen Verbindung und der androgenen Verbindung und die gefäßerweiternde Verbindung gleichzeitig verabreicht werden.

6. Verwendung nach Anspruch 2, wobei die Kombination des Verabreichens der östrogenen Verbindung und der androgenen Verbindung und des Verabreichens der gefäßerweiternden Verbindung in Tablettenform verabreicht wird.

7. Verwendung nach Anspruch 2, wobei die Kombination der östrogenen Verbindung und der androgenen Verbindung auf einer ständigen Basis verabreicht wird und wobei die gefäßerweiternde Verbindung auf einer Leistung-bei-Bedarf-Basis verabreicht wird.

8. Verwendung nach einem der Ansprüche 3 oder 7, wobei die gefäßerweiternde Verbindung vor oder während sexueller Aktivität verabreicht wird.

9. Verwendung nach Anspruch 2, wobei die Kombination der östrogenen Verbindung und der androgenen Verbindung in einer Tablettenform verabreicht wird und wobei die gefäßerweiternde Verbindung in einer Tablettenform verabreicht wird.

10. Verwendung nach Anspruch 2, wobei die Kombination der östrogenen Verbindung und der androgenen Verbindung in einer Tablettenform verabreicht wird und wobei die gefäßerweiternde Verbindung in einer topischen Form verabreicht wird.

11. Verwendung nach Anspruch 10, wobei die topische Form aus der Gruppe bestehend aus Pflastern, Gelen und Cremes ausgewählt ist.

12. Verwendung nach Anspruch 10 oder 11, wobei die topische Form auf die Haut aufgetragen wird.

13. Verwendung nach einem der Ansprüche 10 bis 12, wobei die topische Form auf die Vagina aufgetragen wird.

14. Verwendung nach einem der vorhergehenden Ansprüche, wobei die östrogene Verbindung aus der Gruppe bestehend aus Östron, 17α-Östradiol, 17β-Östradiol, Equilin, 17α-Dihydroequilin, 17β-Dihydroequilin, Equilenin, 17α-Dihydroequilenin, 17β-Dihydroequilenin, Δ^{8,9}-Dehydroöstron, 17α-Δ^{8,9}-Dehydroöstradiol, 17β-Δ^{8,9}-Dehydroöstradiol, Ethinylöstradiol, Östradiolvalerat, 6-OH-Equilenin, 6-OH-17α-Dihydroequilenin, 6-OH-17β-Dihydroequilenin und Mischungen, Konjugaten und Salzen davon ausgewählt ist.

15. Verwendung nach einem der vorhergehenden Ansprüche, wobei die androgene Verbindung aus der Gruppe bestehend aus Methyltestosteron, Androsteron, Androsteronacetat, Androstendion, Androstendiol, Androsteronpropionat, Androsteronbenzoat, Androsterondiol, Androsterondiol-3-acetat, Androsterondiol-17-acetat, Androsterondiol-3,17-diacetat, Androsterondiol-17-benzoat, Androsterondiol-3-acetat-17-benzoat, Androsterondion, Dehydroepiandrosteron, Natriumdehydroepiandrosteronsulfat, Dromostanolon, Dromostanolonpropionat, Ethylöstrenol, Fluoxymesteron, Derdical, Nandrolonphenpropionat, Nandrolondecanoat, Nandrolonfurylpropionat, Nandroloncyclohexanpropionat, Nandrolonbenzoat, Nandroloncyclohexancarboxylat, Oxandrolon, Oxymetholon, Stanozolol, Testosteron, 17-α-Methyl-19-nortestosteron, Testosterondecanoat, 4-Dihydrotestosteron, 5a-Dihydrotestosteron, Testolacton, pharmazeutisch unbedenklichen Estern und Salzen davon und Kombinationen beliebiger der Vorstehenden ausgewählt ist.

16. Verwendung nach einem der vorhergehenden Ansprüche, wobei die gefäßerweiternde Verbindung ein alpha-adrenerger Antagonist ist.

17. Verwendung nach Anspruch 16, wobei die gefäßerweiternde Verbindung Phentolaminmesylat oder Phentolaminhydrochlorid ist.

18. Verwendung nach einem der vorhergehenden Ansprüche, wobei die gefäßerweiternde Verbindung weiterhin Apomorphin enthält.

19. Verwendung nach einem der vorhergehenden Ansprüche, wobei das Arzneimittel weiterhin eine Progestinverbindung umfasst.

20. Verwendung nach Anspruch 19, wobei die Progestinverbindung aus der Gruppe bestehend aus Desogestrel, Dydrogesteron, Ethynodioldiacetat, Medroxyprogesteron, Levonorgestrel, Medroxyprogesteronacetat, Hydroxyprogesteroncaproat, Norethindron, Norethindronacetat, Norethynodrel, Allylöstrenol, 19-Nortestosteron, Lynoöstrenol, Quingestanolacetat, Medrogeston, Norgestrienon, Dimethisteron, Ethisteron, Cyproteronacetat, Chlormadinonacetat, Megestrolacetat, Norgestimat, Norgestrel, Desogestrel, Trimegeston, Gestoden, Nomegestrolacetat, Nomegestrolprogesteron, 5α-Pregnan-3β,20α-diolsulfat, 5α-Pregnan-3β,20β-diolsulfat, 5α-Pregnan-3β-ol-20-on, 16,5α-Pregnen-3β-ol-20-on, 4-Pregnen-20β-ol-3-on-20-sulfat, Acetoxypregnenolon, Anagestonacetat, Cyproteron, Dihydrogesteron, Flurogestonacetat, Gestaden, Hydroxyprogesteronacetat, Hydroxymethylprogesteron, Hydroxymethylprogesteronacetat, 3-Ketodesogestrel, Megestrol, Melengestrolacetat, Norethisteron und Mischungen davon ausgewählt ist.

21. Verwendung nach einem der vorhergehenden Ansprüche, wobei das Arzneimittel dazu formuliert ist, eine schnelle, anhaltende oder verzögerte Freisetzung eines Wirkstoffs bereitzustellen.

22. Verwendung nach Anspruch 9, wobei das Arzneimittel die gefäßerweiternde Verbindung auf einer Basis sofortiger Freisetzung und die östrogene Verbindung und die androgene Verbindung auf einer Basis modifizierter Freisetzung bereitstellt.

23. Verwendung nach einem der vorhergehenden Ansprüche, wobei das Arzneimittel zur Behandlung von weiblicher sexueller Dysfunktion in einer postmenopausalen Frau ist.

## Revendications

1. Utilisation d'un composé oestrogénique, d'un composé androgénique et d'un composé de vasodilatation pour la fabrication d'un médicament oral pour le traitement d'un dysfonctionnement sexuel féminin.

2. Utilisation d'un composé oestrogénique, d'un composé androgénique et d'un composé de vasodilatation pour la fabrication de médicaments pour le traitement d'un dysfonctionnement sexuel féminin, dans laquelle le composé oestrogénique et le composé androgénique sont combinés comme premier médicament oral et le composé de vasodilatation est fourni comme second médicament.

3. Utilisation selon la revendication 2, dans laquelle le composé de vasodilatation est administré en fonction des performances à la demande.

4. Utilisation selon la revendication 2, dans laquelle la combinaison du composé oestrogénique et du composé androgénique est administrée de manière chronique et dans laquelle le composé de vasodilatation est administré de manière chronique.

5. Utilisation selon la revendication 2, dans laquelle la combinaison du composé oestrogénique et du composé androgénique et le composé de vasodilatation sont administrés simultanément.

6. Utilisation selon la revendication 2, dans laquelle la combinaison d'administration du composé oestrogénique et du composé androgénique et d'administration du composé de vasodilatation est administrée sous forme de comprimé.

7. Utilisation selon la revendication 2, dans laquelle la combinaison du composé oestrogénique et du composé androgénique est administrée de manière chronique et dans laquelle le composé de vasodilatation est administré en fonction des performances à la demande.

8. Utilisation selon l'une quelconque de la revendication 3 ou 7, dans laquelle le composé de vasodilatation est administré avant ou au cours de l'activité sexuelle.

9. Utilisation selon la revendication 2, dans laquelle la combinaison du composé oestrogénique et du composé androgénique est administrée sous forme de comprimé et dans laquelle le composé de vasodilatation est administré sous forme de comprimé.

10. Utilisation selon la revendication 2, dans laquelle la combinaison du composé oestrogénique et du composé androgénique est administrée sous forme de comprimé et dans laquelle le composé de vasodilatation est administré sous forme topique.

11. Utilisation selon la revendication 10, dans laquelle la forme topique est sélectionnée parmi le groupe constitué de timbres transdermiques, gels et crèmes.

12. Utilisation selon la revendication 10 ou la revendication 11, dans laquelle la forme topique est appliquée à la peau.

13. Utilisation selon l'une quelconque des revendications 10 à 12, dans laquelle la forme topique est appliquée au vagin.

14. Utilisation selon l'une quelconque des revendications précédentes, dans laquelle le composé oestrogénique est sélectionné parmi le groupe constitué de l'oestrone, du 17α-oestradiol, du 17β-oestradiol, de l'équiline, de la 17α-dihydroéquiline, de la 17β-dihydroéquiline, de l'équilénine, de la 17α-dihydroéquilénine, de la 17β-dihydroéquilénine, de la Δ^{8,9} -déhydrooestrone, du 17*α-Δ*^{8,9} -déhydrooestradiol, du 17β-Δ^{8,9} -déhydrooestradiol, de l'éthinyloestradiol, du valérate d'oestradiol, de la 6-OH équilénine, de la 6-OH 17α-dihydroéquilénine, de la 6-OH 17 β-dihydroéquilénine et de mélanges, conjugués et sels de ceux-ci.

15. Utilisation selon l'une quelconque des revendications précédentes, dans laquelle le composé androgénique est sélectionné parmi le groupe constitué de la méthyltestostérone, de l'androstérone, de l'acétate d'androstérone, de l'androsténédione, de l'androsténédiol, du propionate d'androstérone, du benzoate d'androstérone, de l'androstéronediol, de l'androstéronediol-3-acétate, de l'androstéronediol-17-acétate, de l'androstéronediol-3,17-diacétate, de l'androstéronediol-17-benzoate, de l'androstéronediol-3-acétate-17-benzoate, de l'androstéronédione, de la déhydroépiandrostérone, du sulfate sodique de déhydroépiandrostérone, de la dromostanolone, du propionate de dromostanolone, de l'éthylestrénol, de la fluoxymestérone, du phénopropionate de nandrolone, du décanoate de nandrolone, du furylpropionate de nandrolone, du cyclohexane-propionate de nandrolone, du benzoate de nandrolone, du cyclohexanecarboxylate de nandrolone, de l'oxandrolone, de l'oxymétholone, du stanozolol, de la testostérone, de la 17α-méthyl-19-nortestostérone, du décanoate de testostérone, de la 4-dihydrotestostérone, de la 5α-dihydrotestostérone, de la testolactone, des esters et sels pharmaceutiquement acceptables de ceux-ci, et des combinaisons de l'un quelconque des composés précédents.

16. Utilisation selon l'une quelconque des revendications précédentes, dans laquelle le composé de vasodilatation est un antagoniste alpha-andrénergique.

17. Utilisation selon la revendication 16, dans laquelle le composé de vasodilatation est le mésylate de phentolamine ou le chlorhydrate de phentolamine.

18. Utilisation selon l'une quelconque des revendications précédentes, dans laquelle le composé de vasodilatation comprend en outre de l'apomorphine.

19. Utilisation selon l'une quelconque des revendications précédentes, dans laquelle le médicament comprend en outre un composé de progestine.

20. Utilisation selon la revendication 19, dans laquelle le composé de progestine est sélectionné parmi le groupe constitué du désogestrel, de la dydrogestérone, du diacétate d'éthynodiol, de la médroxyprogestérone, du lévonorgestrel, de l'acétate de médroxyprogestérone, du caproate d'hydroxyprogestérone, de la noréthindrone, de l'acétate de noréthindrone, du noréthynodrel, de l'allylestrénol, de la 19-nortestostérone, du lynoestrénol, de l'acétate de quingestanol, de la médrogestone, de la norgestriénone, de la diméthistérone, de l'éthistérone, de l'acétate de cyprotérone, de l'acétate de chlormadinone, de l'acétate de mégestrol, du norgestimate, du norgestrel, du désogestrel, de la trimégestone, du gestodène, de l'acétate de nomégestrol, de la progestérone de nomégestrol, du sulfate de 5α-prégnan-3β,20α-diol, du sulfate de 5α-prégnan-3β,20β-diol, de la 5α-prégnan-3β-ol-20-one, de la 16,5α-prégnén-3β-ol-20-one, du 4-prégnén-20β-ol-3-one-20-sulfate, de l'acétoxyprégnénolone, de l'acétate d'anagestone, de la cyprotérone, de la dihydrogestérone, de l'acétate de flurogestone, du gestadène, de l'acétate d'hydroxyprogestérone, de l'hydroxyméthylprogestérone, de l'acétate d'hydroxyméthylprogestérone, du 3-cétodésogestrel, du mégestrol, de l'acétate de mélengestrol, de la noréthistérone et de mélanges de ceux-ci.

21. Utilisation selon l'une quelconque des revendications précédentes, dans laquelle le médicament est formulé pour fournir une libération rapide, soutenue ou retardée d'un ingrédient actif.

22. Utilisation selon la revendication 9, dans laquelle le médicament fournit le composé de vasodilatation en fonction d'une libération immédiate et le composé oestrogénique et le composé androgénique en fonction d'une libération modifée.

23. Utilisation selon l'une quelconque des revendications précédentes, dans laquelle le médicament est pour le traitement d'un dysfonctionnement sexuel féminin chez une femme post-ménopausale.
